Europäisches Patentamt

European Patent Office

Office européen des brevets

(11) Publication number: **0 508 319 A2**

# EUROPEAN PATENT APPLICATION

(21) Application number: **92105785.7**

(51) Int. Cl.5: **G01N 33/48**

(22) Date of filing: **03.04.92**

(30) Priority: **08.04.91 DE 4111317**

(43) Date of publication of application:
**14.10.92 Bulletin 92/42**

(84) Designated Contracting States:
**DE ES FR GB IT**

(71) Applicant: **PALL CORPORATION**
**30 Sea Cliff Avenue**
**Glen Cove New York 11542(US)**

(72) Inventor: **Heusslein, Ralph, Dr.**
**Im Wingertsgrund 66**
**W-6374 Steinbach/Taunus(DE)**

(74) Representative: **Dost, Wolfgang, Dr. rer. nat.**
**Dipl.-Chem. et al**
**Patent- u. Rechtsanwälte Bardehle,**
**Pagenberg, Dost, Altenburg Frohwitter &**
**Partner Galileiplatz 1**
**W-8000 München 80(DE)**

(54) Method of evaluating pathological conditions which involve a change in the composition of body fluids.

(57) In a method of evaluating pathological conditions, which involves a change in the composition of body fluids, an isolated nerve is initially conditioned in standard electrolyte solution and then a measuring chamber is alternately supplied with a calibration fluid and a test fluid. One then allows a stimulus impulse to act on the nerve by means of electrodes in contact with the nerve and measures the action potential of the nerve actuated by the stimulus impulse for the concerned fluid.

The evaluation of the pathological conditions is performed according to the differences in the action potentials between calibration fluid and test fluid or between various test fluids.

Fig. 2

EP 0 508 319 A2

A change in the composition of body fluids can often be observed, for example with respect to protein content and cell count of the cerebrospinal fluid, in various neurological diseases, such as Guillain-Barré Syndrome (GBS), Multiple Sclerosis (MS), Paraproteinosis or Radikulitidis, which manifest themselves, among other things, by a change of the impulse transmission in nerves. The pathogenesis of these diseases remains largely unclarified. It has been shown that immunologically induced reactions lead to degenerative processes on the myelin sheaths and/or on the axons, which in turn cause a delayed impulse transmission in the nerves or lead to a reduction in conductivity or in the extreme case, block the conduction. The consequences are extensive disruptions in the motor and/or sensory functions, which in grave cases can lead to complete paralysis of the patients, so that they can only be kept alive by artificial respiration.

Currently the therapeutic treatment of GBS consists mainly of a plasmapheresis (partial plasma exchange) and/or the administration of medications which suppress the immune response of the patient, such as glucocorticoids or other immunosuppressants. Most recently, the administration of immunoglobulins has also been considered as a therapeutic approach. All of the mentioned therapeutic treatments, however, involve considerable drawbacks.

A completely new therapeutic approach in the treatment of GBS consists of the filtration of the cerebrospinal fluid using special filters (see EP 90119187.4). The results of this treatment show that the composition of liquor cerebrospinalis is apparently of great significance, since a distinct improvement in the symptoms of the patient can be determined already in a relatively short time (hours to days) after performing the filtration treatment.

To date, however no possibility exists in the study of GBS and other neuropathies for evaluating the acute pathological symptoms or the measures taken in therapeutic treatment of the neuropathies, for example an evaluation of the filters used in filtration of the liquor cerebrospinalis. An animal model exists as the so-called EAN-mouse, whereby degenerative, demyelinating effects are observed on the mouse nerves after injection of human body fluid, for example serum or liquor cerebrospinalis, which correspond to the symptoms of GBS. This animal model, however is hardly suitable for evaluating pathological conditions for a number of different reasons.

An abstract of the lecture "blocking effect of the Guillain-Barré-liquor on the nervus ischiadicus of the rat" by Hülser et. al. on the symposium of the Deutschen Gesellschaft für Neurologie in Bad Nauheim from September 29, 1989 to October 1, 1989, reports on investigations of liquor cerebrospinalis of patients with GBS. Herewith, 50 $\mu l$ liquor were injected subperineurally, i.e. intraneurally, into the right or left nervus ischiadicus of a rat, respectively. After distale stimulation of the nervus tibialis, lumbal somato sensible evoked potentials were recorded before the injection, and 5 or 20 minutes, respectively after the injection. Herewith, a conduction block was observed after injection of liquor which has been taken before liquor filtration, whereas the conduction block did not appear after liquor filtration (liquorpheresis). Hence, it is concluded that factores are removed by the liquor filtration, which are responsible for the conduction block by the Polyradikulitidis or GBS, respectively.

This abstract refers to a generally known standard method for the measurment of nerv action potentials by using an anesthesized rat, wherein the concerning nerv is laied open before the measuring. Disadvantages of this method consists therein that, as a consequence of the relative great amount of injected liquide (formation of local odemes) and operative changes, impairment or damage, respectively of the nerve were caused which makes the results unreproducible. A further disadvantage of the method results therein that one test animal is immolated for every investigation of a patient.

Because of the disadvantages of said method for measuring action potentials, from the first said method was less suitable as a measuring technique for a commercial suitable and, especially, reproducible method for evaluating neurological pathological conditions. However, it was surprising that said method can yet serve as a starting point for such a procedure, when one measures with an isolated nerv instead of an anesthesized test animal, and does not inject the body fluid to evaluat into the nerv, rather the nerv was surrounded or sprinkled, respectively by the body fluid.

The invention concerns, consequently, to a method for evaluating neurological pathological conditions, which involves a change in composition of body fluids, wherein a nerve is exposed to the body fluid, the action potential of the nerve is measured, and an evaluation of the pathological conditions is made based on the potential differences before and after the exposure of the nerve to the body fluid, characterized in that an isolated nerve is used and the body fluid is not intraneurally injected, rather the nerve is wetted by the body fluid.

It was surprising that the action potential of the isolated nerves, i.e. nerve potential change within time produced by the stimulating impulse, adjusted rapidly and reproducibly after each fluid exchange in the measuring chamber and that also, after one had subjected the isolated nerve to a pathogenic body fluid, the action potential, which varied because of the pathogenic fluid, rapidly returned to the normal action potential after replacing the pathogenic fluid with the normal fluid, i.e. for example a physiological electrolyt solution. In other words, the observed variations in the action potentials are completely reversible, at least within the scope of the described evaluation method. The reversibility is, therefore, surprising because with respect to GBS, MS and other neuropathies, one had discussed a degenerative damage of the nerves, i.e. anatomic alterations. Saida et. al., Laboratory Investigation 39 (1978) 449-A62, reports that already 20 minutes after injection of EAN serum into the nervus ichiaticus of a test animal irreversible degenerative damages of the nerv appeared, which was confirmed by electron microscopic investigations. So, one could not have expected that rapide and reproducible evaluation of the mentioned pathological conditions is possible.

Isolated nerves are used in performing the method according to the present invention. An isolated nerv is concerned to the axonal segments of nerv cells; dentrites and somas are therefore removed. Basically there are no limitations, the method however is preferably performed on isolated nervus ischiadicus of mice or on spinal nerv roots of cattle.

In the case of mice, the animal is killed without using pharmacals, for example by breaking its neck, directly before preparation of the mouse nerves. The nerves (nervus ischiadicus) are carefully prepared in their entire length from the beginning in tissue up to the spine, whereby the epineurium is preferably retained for reasons of practicality. A mechanical impairment of the nerves, for example by squeezing or stretching is to be absolutely avoided. During and after the preparation, the nerves lie in a bath of physiological electrolyte solution at a temperature of about 30°C.

In a preferred embodiment of the method according to the present invention the spinal nerves of cattle are used, wherein from freshly slaughtered cattle, which were divided along the spine, 4 to 5 cm long pieces in the area of $L_1$, $L_2$ and $L_3$ between spinal marrow and dura mater were cutted off. Therefore, it is concerned to a bundle of fascicles which can be easily separated. The separation of the single nerves took place by cutting off before the actual measurement. From the taking of the nerve from the slaughtered cattle, the nerves were stored permanently in a physiological electrolyte solution (containing glucose) at 20 to 38°C, preferably at 30°C. It was surprising that the life-span of the cattle nerves is diminished at low temperatures. In this way 10 to 30 fascicles per spinal nerve root can be obtained. The obtained cattle nerves are functionable from 8 to 10 hours, when the physiological electrolyte solution with the nerve has access to oxygen.

The instruments used for preparation and manipulation of the nerves are stored in electrolyte solution to reduce the release of metal ions.

Basically the method according to the invention can be carried out with a single isolated nerve. Preferably, however, two identical nerves or nerve sections are used for reasons of measuring accuracy and reproducibility.

A preferred embodiment of the invention is characterized in that one
- conditions the isolated nerve under standard electrolyte solution,
- supplies a measuring chamber alternatively with a calibration fluid and a test fluid,
- introduces a stimulating impulse into the nerve by means of electrodes in contact with the nerve and measures the action potential of the nerve induced by the stimulating impulse for the concerned fluid, and performs the evaluation according to the differences between the action potentials of the calibration fluid and the test fluid or between various test fluids.

Thereby the nerve is arranged in the center of a measuring chamber provided with opposing openings for the nerve, such that sufficient nerve portions are still available on both sides of the measuring chamber for introducing the stimulating impulse and recording the action potentials. Several electrodes are attached extracellularly to the projecting nerve portions respectively for stimulating the nerve and recording the action potentials. The nerve is preferably placed upon a previously fixed electrode arrangement due to the sensitivity of the nerve with respect to the smallest changes. The electrode arrangement on each side of the measuring chamber consists of a plurality, for example five each, of thin wires arranged at a spacing to one another (for example about 1mm spatial separation), preferably of platin, for example 0.6mm diameter. The electrodes suited for the desired measurement according to position and number are then selected from these electrodes by making electrical contact. For stimulation, one uses for example two electrodes at a spacing of about 1mm (free space separation), between which the stimulating current flows. An anodic stimulus is preferred due to the low threshold voltage and the approximation to the physiological situation. A third electrode is of advantage for practical reasons, which is connected to ground potential.

After one has confirmed based on the potentials that the nerve is intact and a trouble-free contact to the electrodes exists, the measuring chamber is sealed at the openings, for example with silicone grease, and then filled with a standard electrolyte solution, so that the nerve is surrounded with electrolyte solution in the measuring chamber section. The nerve portions extending on both sides from the measuring chamber are then placed under wet chamber conditions, i.e. one creates closed chambers with water vapor saturation above standard electrolyte solution. This can be accomplished, for example, in a simple manner by introducing cellulose material soaked with standard electrolyte solution into the closed sections. The measuring chamber is provided with an oxygen supply. The oxygen supply to the nerve serves to maintain the metabolic processes (for example $K^+$-$Na^+$ pump), to maintain the functionality of the nerve as long as possible. In practice, this can be provided for example by controlled feed of warmed and humidified air. Through this, the fluid in the measuring chamber is simultaneously mixed, so that concentration deviations within the measuring chamber are substantially avoided. The measuring chamber is made of physiologically neutral material, for example glass or synthetic substances.

Two electrodes are used on the recording side employing differential recording. For simple recording, a single electrode would be sufficient (measurement against ground). An electrode spacing of about 2.5mm (spatial separation) is advantageous for differential recording.

The signals recorded from the recording electrodes are amplified in a manner known per se. A differential amplifier, for example, is used for this. According to definition, an action potential is the potential of a single axon. For a nerve having a bundle of axons, the sum of the action potentials are recorded as the so-called sum action potential. For using this recording technique, three parameters are preferably determined, namely,

1. the maximal amplitude of the measured potential,

2. the time relative to the start of stimulation at which this maximum is reached (latency), and

3. the area under the curve of the sum action potential.

Before the actual measuring phase, a conditioning takes place, i.e. the entire system is placed in a water bath, preferably with a high temperature-accuracy (for example 37.5°C + 0.1°C, short response times), long enough (in general about 1.5 hours) that the system stabilizes, i.e. until no signifcant time-dependance of the measurement parameters is observed.

The body fluids principally include plasma, serum, and liquor cerebrospinalis. In the following, the operation of the method of the present invention is described by way of example with liquor cerebrospinalis.

First of all, the nerve is conditioned with standard electrolyte solution, which in its composition substantially corresponds to the electrolyte content and the osmolarity of liquor cerebrospinalis (for example, electrolyte solution HF23, available from Fresenius AG, Germany). At the beginning of the conditioning phase, the electrolyte solution is exchanged several times. This conditioning phase (about 1.5 hours) provides for stabilization of the measurement parameters.

Subsequently, the electrolyte solution is drawn out of the measuring chamber and replaced by the liquor cerebrospinalis to be studied. Here, it is advantageous to carry out two parallel runs with identical nerves or nerve sections, where one chamber is filled with the liquor to be studied and the other chamber is filled with the same liquor; however, which has previously been subjected to a treatment, for example a filtration for removal of likely pathogenic components. For all manipulations with the measuring chamber and in particular when suctioning off and filling the measuring fluid, care should be taken that the nerve is not contacted or moved, since this can influence the stimulation or recording from the nerve due to changes in the electrode contact.

The actual measuring phase lasts about one to two hours. Following this, the reversibility of the measured phenomenon is controlled, where the measuring chamber is either filled with the filtered liquor or with the standard electrolyte (HF23) depending on the starting situation. This phase also serves as a control of whether the nerve responds differently than it did at the beginning of the run.

The above-mentioned three measuring parameters, i.e. the maximal potential height, latency and the sum action potential area, are calculated and stored in a microcomputer system throughout the entire duration of the run, for example at five minute intervals. The time-course of the parameters are later analyzed in relative terms, i.e. the values determined at the start of the run in the conditioning phase with standard electrolyte solution are taken as the standard (= 100%).

The preparation of the liquor fluid taken from various patients at the present time is as follows: directly after punctuation of the liquor volume, the liquor is centrifuged (about 400 rpm) for about 5 minutes for removal of cells. The topping is decanted and either freshly processed or stored, for example at -80°C. A portion of the liquor is filtered before beginning the run (for example 1ml filtrate). The flow across the membrane used for filtration is held constant to guarantee the reproducibility of the tests. Parallel to this, a comparable amount (about 1ml) of the same starting solution is also kept at room temperature, and the two samples are tempered before beginning the tests for about 0.5 hours in a water bath (for example at 37.5°C).

The invention is described in the following in conjunction with the drawing.

Fig. 1 shows a schematic drawing of an arrangement for carrying out the method of the invention.

Fig. 1a shows a plan view of a synthetic material block 1 of Fig. 1.

Fig. 1b shows a longitudinal section (as indicated in Fig. 1a) through the synthetic material block 1.

Fig. 1c shows a cross-section (as indicated in Fig. 1a) through the synthetic material block 1.

Fig. 2 shows a typical potential curve recorded from nervus ischiadicus of a mouse. The ordinate is not numbered. $V_{max}$ corresponds to about 5mV.

Fig. 3 shows the reaction of the nervus ischiadicus of the mouse (sum action potential) to liquor cerebrospinalis of a neurologically healthy patient. The area under the sum action potential is illustrated as an indicator for the total activity of the nerve (related to the standard at the conditioning phase = 100%).

Fig. 4 shows, in the same manner of illustration as in Fig. 3, the reaction of nervus ischiadicus of the mouse to the liquor of a GBS patient, in one case untreated (native) and in the other case filtered (filtrate).

Fig. 5 shows, in the same manner of illustration as in Fig. 3, the reaction of nervus ischiadicus of the mouse under the influence of liquor cerebrospinalis of a GBS patient in the time-sequence: standard electrolyte solution - liquor cerebrospinalis - liquor cerebrospinalis filtered - standard electrolyte solution.

Fig. 6 shows the reaction of nervus ischiadicus of the mouse to liquor cerebrospinalis of a GBS patient, in one case untreated (native) and in the other case filtered (filtrate).

Fig. 7 shows, in the same manner of illustration as in Fig. 3, the reaction of spinal nerv roots of cattle to the liquor cerebrospinalis of an GBS patient, in one case untreated (native, indicated with filled rombs) and in the other case filtered (filtered, indicated with open squares).

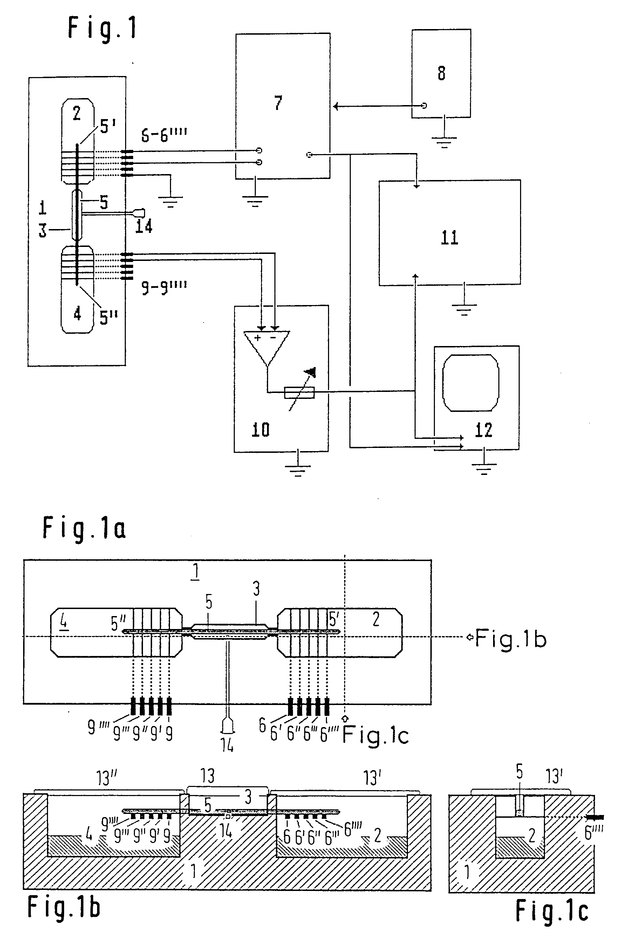

The Figs. 1, 1a, 1b and 1c show a schematic diagram of an arrangement for carrying out the method according to the present invention. Three chambers 2, 3 and 4 are located in a synthetic material block 1, which were produced by milling. The outer two chambers 2, 4 are larger than the central measuring chamber 3. A nerve 5 is arranged in the measuring chamber 3 so that projecting nerve ends 5', 5'', remain on both sides of the measuring chamber 3 for receiving stimulation impulses and the recording of sum action potentials. For this purpose, electrodes 6, 6', 6'', 6''' and 6'''' are located on a stimulation side in the chamber 2 in the form of thin platin wires (diameter 0.6mm).

Electrical stimulation impulses at a frequency of 4Hz with a duration of 1msec and an intensity of 2V are applied the nerve 5 via two electrodes 6', 6'' (or another combination of electrodes), by means of a stimulus generator 7 and a timer 8 every 5 minutes for 30 seconds respectively. The electrode 6 located adjacent to the measuring chamber 3 is connected to ground. On the recording side, the electrodes 9, 9', 9'', 9''' and 9'''' are arranged in the other chamber 4, also made of platin wire and also arranged as on the stimulation side. The further electrodes 9', 9'', 9''' and 9'''' are provided for leading off the sum action potential. The illustrated differential recording with two electrodes (with selectable spacing of the electrode 9' to one of the electrodes 9'', 9''' and 9'''' by means of selectable electrical contacting) and a differential amplifier 10 is advantageous.

The nerve 5 is arranged in the synthetic material block 1 so that it lies stably in the center on the floor of the measuring chamber 3 and at both sides on the electrodes 6, 6', 6'', 6''', 6'''', 9, 9', 9'', 9''' and 9'''' (see Figs. 1a to 1c) and so that a stable sum action potential is recorded. An oscilloscope 12 is provided for optimal representation of the fast voltage changes, and parallel to this, the signals are stored 11 analogue or digitally and analyzed on- or off- line. A pulse (trigger) of the stimulus generator 7 at the start of a stimulation is used as a reference-point for the latency of the potential.

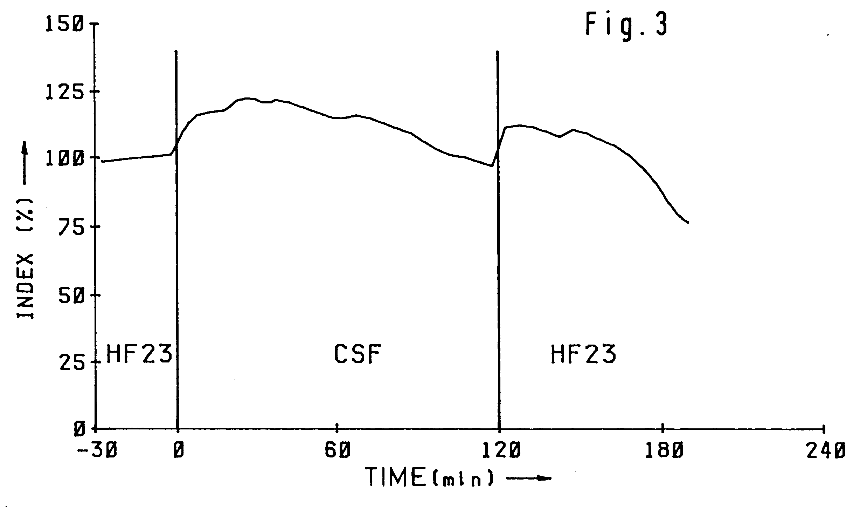

After the stabilization of the sum action potential has been checked, the nerve is sealed by means of silicone grease at the openings of the measuring chamber 3 to the chambers 2, 4, so that the measuring chamber 3 to be filled with fluid does not leak. These steps must be performed as fast as possible (< 5 minutes), since the nerve at this time is not in electrolyte solution. By filling standard electrolyte into the measuring chamber 3, the nerve lies in this section in electrolyte solution. Standard electrolyte solution is also filled into the outer chambers 2, 4. It, however, may not wet the electrodes under any circumstances. In practice, this is achieved by milling out the chambers 2 and 4 deeply (see Fig. 1b), which makes it possible to apply a fleece wetted with electrolyte to the floor of the chambers, which does not touch the electrodes. The two outer chambers 2, 4 are then each immediately closed with a plastic platelet 13', 13'' (for example, Teflon), so that two moist chambers are formed with water vapor saturated atmosphere. The measuring chamber 3 is covered with a plastic platelet 13 so that the air in the test solution fed through the cannula 14 can escape. The nerve is conditioned (about 1.5 hours) by repeated exchange of the standard electrolyte solution in the measuring chamber 3. Afterwards, the electrolyte solution is replaced by the solution to be tested. The continued measurement of the sum action potential during the entire test duration is analyzed at the end of the test (see Figs. 3 to 7). Individual fluctuations of the action potentials of individual measurements are compensated by averaging several individual measurements.

Fig. 1a is a plan view of the synthetic material block 1 of Fig. 1.

Fig. 1b shows a longitudinal section (as indicated in Fig. 1a) through the synthetic material block 1, and Fig. 1c shows a cross-section (as indicated in Fig. 1a) through this synthetic material block 1.

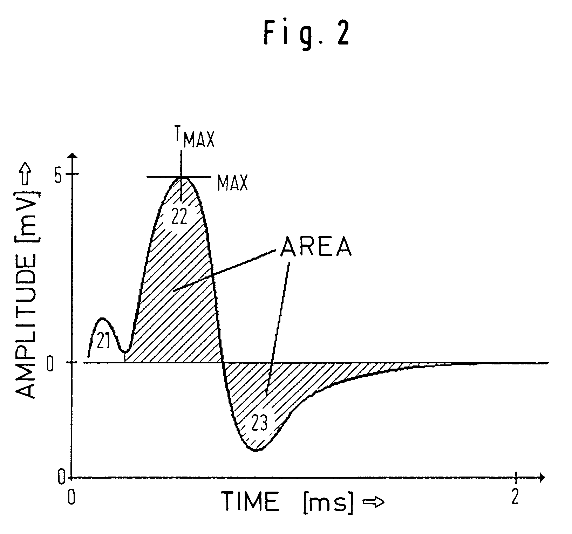

The typical time-course of a sum action potential is illustrated in Fig. 2. A short potential increase 21 during the first 0.2 msec (200 $\mu$sec) arises due to the electrical conduction of the stimulus (so-called stimulus artefact). A sharp voltage increase 22 thereafter and a drop-off 23 to negative values is typical for a differential recording. The entire sum action potential last about 2 msec. The shape and time-course of the potential can vary with different electrode spacings. Three parameters which characterize the potential curve are determined. These is the maximum amplitude, MAX (mV), the time from starting the stimulus until reaching this maximum, $T_{max}$ (msec), and the area under the curve (mV x msec), where the stimulus artefact may not be included in calculating the area. Especially the area under the curve has turned out to be a very good measure parameter for the total activity of the axons in a nerve.

Fig. 3 shows the area (index) under the sum action potential (see Fig. 2) obtained for nervus ischiadicus of the mouse in relative units as a function of time. The values measured in the conditioning phase are set to 100%. At the time 0, a standard electrolyte solution (HF23) is replaced by cerebrospinal fluid (CSF) of a healthy patient. The cerebrospinal fluid (CSF) is again exchanged with standard electrolyte HF23 after 2 hours. The represented behavior of the measuring parameter is typical. The presented changes of the solutions also occur in a similar manner when standard electrolyte is replaced by standard electrolyte.

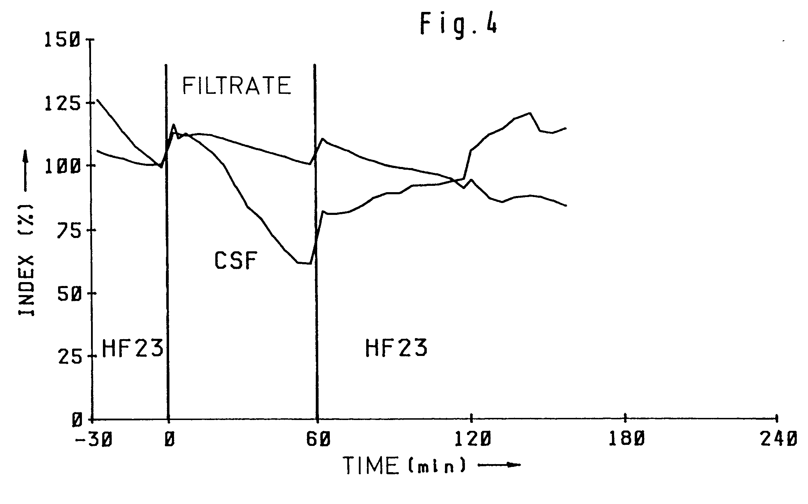

A test with cerebrospinal fluid (CSF) of a GBS patient is represented in Fig. 4 in the same manner as in Fig. 3. While the curve obtained with the filtered cerebrospinal fluid (filtrate) corresponds to that of Fig. 3, i.e. that of a healthy patient, a reduction of the area under the sum action potential is found for the non-filtered cerebrospinal fluid (CSF) as an indication that the activity of the axons was reduced. After washing with standard electrolyte (HF23), the measured effect is fully reversible after 1 hour.

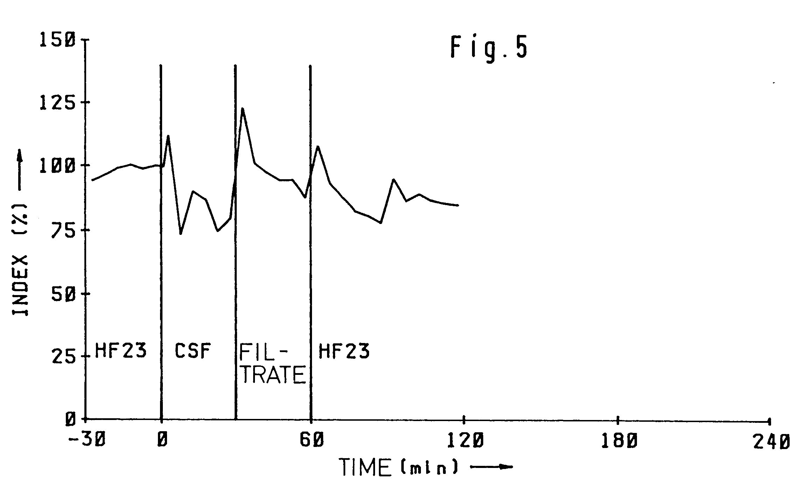

In Fig. 5, in the same presentation as in Fig. 3, conditioning is performed initially with standard electrolyte (HF23). At time 0, the fluid in the measuring chamber is replaced by cerebrospinal fluid (CSF) of a GBS patient. A reduction of the area under the sum action potential is found. After 30 minutes the cerebrospinal fluid (CSF) is suctioned off and replaced by CSF filtrate (filtrate). It is found that the area under the sum action potential now returns to the values measured in the conditioning phase with HF23. Replacement with standard electrolyte (HF23), shows that the measured values remain at the normal level.

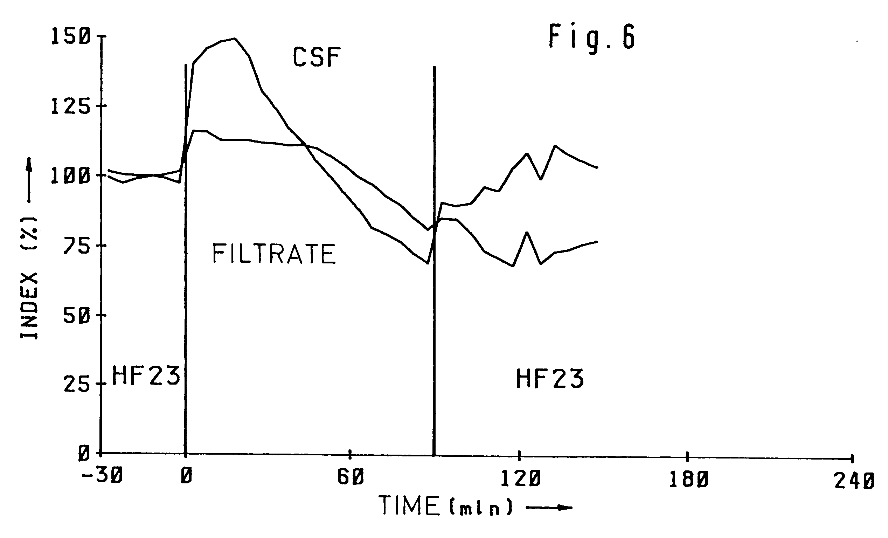

Fig. 6, in the same presentation as in Fig. 3, shows that the filtrate here of cerebrospinal fluid (filtrate) of a GBS patient on a mouse nerve also has about the same effect as cerebrospinal fluid of a healthy patient (see Fig. 3). It is distinctly seen, however; that for this GBS patient, the unfiltered cerebrospinal fluid (CSF) causes an excessive activity. Immediately after changing the solution of standard electrolyte HF23 to cerebrospinal fluid (CSF), an increase in activity of about 50% is measurable.

However, this effect is maintained only for a short time, it is not observed for the filtered cerebrospinal fluid (filtrate). The observed behavior and the differences with respect to Fig. 5, can be explained by the selective influence on various ion channels in the axon membranes.

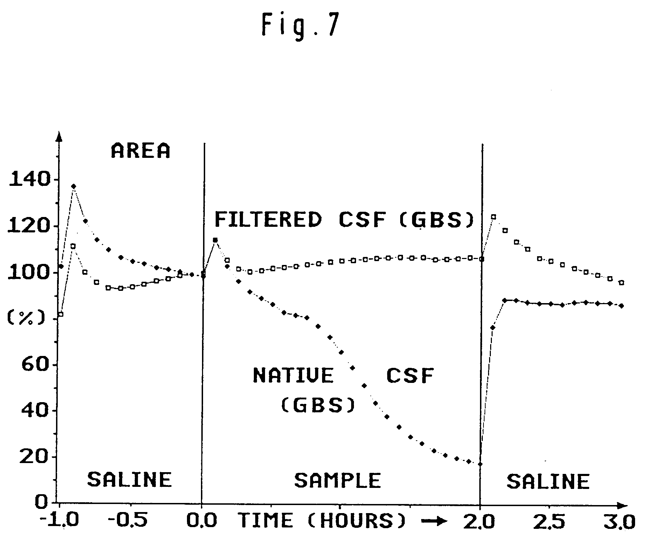

Fig. 7, in the same presentation as in Fig. 3, shows the reaction of a fascicle spinal nerv root of cattle on cerebrospinal fluid of a GBS patient. After the conditioning phase (HF23), one observes a distinct difference between filtered cerebrospinal fluid (squares) and unfiltered cerebrospinal fluid (rombs) in their effect on the cattle nerve. After over two hours, these effects are completely reversible after washing with standard electrolyte (HF23).

## Claims

1. A method for evaluating neurological pathological conditions, which involves a change in composition of body fluids, wherein a nerve is exposed to the body fluid, the action potential of the nerve is measured, and an evaluation of the pathological conditions is made based on the potential differences before and after the exposure of the nerve to the body fluid, **characterized** in that an isolated nerve is used and the body fluid is not intraneurally injected, rather the nerve is wetted by the body fluid.

2. Method of claim 1, characterized in that one uses an isolated mouse nerve.

3. Method of claim 2, characterized in that one uses the nervus ischiadicus.

4. Method of claim 1, characterized in that one uses an isolated cattle nerve.

5. Method of claim 4, characterized in that one uses the spinal nerve root.

6. Method according to one of claims 1 to 5, characterized in that liquor cerebrospinalis is used as body fluid.

7. Method of claim 6, characterized in that one performs an evaluation of Guillian-Barré Syndrome (GBS) or Multiple Sclerosis (MS).

8. Method according to one of the preceding claims, characterized in that one
   - conditions the isolated nerve in standard electrolyte solution,
   - alternately supplies a measuring chamber with calibration fluid and test fluid,
   - allows a stimulus impulse to act on the nerve by means of electrodes in contact with the nerve and measures the action potential of the nerve initiated by the stimulus impulse for the concerned fluid,
   - and performs the evaluation according to differences in the action potentials between calibration fluid and test fluid or between various test fluids.

# Fig.1

# Fig.1a

# Fig.1b

# Fig.1c

# Fig. 2

Fig.3

Fig.4

FILTRATE

CSF

HF23

HF23

INDEX (%) ——►

150
125
100
75
50
25
0

-30     0        60        120       180       240
TIME (min) ——►

EP 0 508 319 A2

Fig.5

Fig. 6

EP 0 508 319 A2

# Fig.7